# EUROPEAN PATENT SPECIFICATION

(11) **EP 4 392 112 B1**
(45) Date of publication and mention of the grant of the patent: **14.10.2026**
(21) Application number: 22860784.2
(22) Date of filing: 21.08.2022
(51) Int. Cl.: A61M 25/00, A61M 39/00, A61M 39/22

(54) **PERIPHERALLY INSERTED CENTRAL CATHETERS (PICC)**
PERIPHER EINGEFÜHRTE ZENTRALKATHETER (PICC)
CATHÉTERS CENTRAUX À INSERTION PÉRIPHÉRIQUE (PICC)

(30) Priority: 24.08.2021 US 202163236538 P
(43) Date of publication of application: 03.07.2024
(73) Proprietor: Shiri, Alon, 5126107 Bnei-Brak (IL)
(72) Inventor: BARNEA, Yaacov, Singapore 238874 (SG)
(74) Representative: Kancelaria Eupatent.pl Sp. z o.o.
(86) International application number: PCT/IL2022/050912
(87) International publication number: WO 2023/026279

(56) References cited:
- WO-A1-2004/041343
- WO-A1-2014/092898
- WO-A1-2017/053882
- CN-A- 105 879 202
- US-A1- 2005 261 663
- US-A1- 2009 054 874
- US-A1- 2013 289 527
- US-A1- 2017 072 129
- US-A1- 2018 042 623
- US-B1- 10 099 009

## Description

The present disclosure relates generally to Peripherally Inserted Central Catheters (PICC) and methods of use (methods not claimed) thereof

### BACKGROUND

A catheter is a long-established medical device based on a hollow lumen generally introduced into a body organ or a blood vessel for draining fluids, delivery of medical substances, nutrition, monitoring and other medical-related procedures. Peripherally Inserted Central Catheters (PICC) is a family of catheters typically implemented for a long-term medical intravenous (IV) therapy, such as cancer treatment. Generally, the insertion of the PICC is performed via a peripheral arm vein using nitinol guidewire and X-ray imaging. Inserted PICC devices may be remained inside a patient's body for a prolonged period in some cases of more than 6 months. Such a long duration of usage adds to the risk of PICC causing infections, theses may include external bacteria dwelling into the body, for instance, during the administration of the device, and/or due to gap presence between the PICC and the body, internal bacteria or contaminations presence. Catheter-related bloodstream infections, bacteria growth, as well as blood clotting, occlusion, precipitates deposition, air embolism, disposition, leakage, kinks presence and other catheter malfunctions are highly associated with deterioration of the patient's condition and may even lead to mortality.

U.S. Patent Application 2013/0289527 A1 discloses a catheter device.

Flushing of the PICC device with a cleaning solution, such as saline, is a commonly applied method performed routinely, also following medical treatments, for removing undesired substances and bacteria from the catheter lumen and maintaining the catheter patency. Adequately performed flushing procedure is of great importance for preventing the accumulation of the undesired substances inside the catheter lumen.

Nevertheless, there is a need in the art for an improved PICC design and flushing method for facilitating disinfecting and removing the undesired substances from the catheter lumen.

### SUMMARY

The aforementioned objectives are achieved according to the invention by means of a peripherally insertable central catheter (PICC) according to claim 1. Preferred embodiments are defined in the dependent claims.

The following description includes references to methods of use and/or of flushing of the inventive device, these methods not falling under the invention as claimed.

Aspects of the disclosure, according to some embodiments thereof, relate to a catheter, and specifically but not exclusively to a peripherally insertable central catheter (PICC) for administration fluids, such as medical substances and nutrition, into the subject. Following the delivery of the medical substances into a blood vessel and/or body organ, the PICC is regularly cleaned by introducing a flushing fluid, such as saline, into the catheter. Although the flushing fluid minimizes accumulation of bacteria, clotting factors, and the like, inside the catheter, the flushing fluid is typically drained into the subject's body, administrating undesired substances into the subject. However, the special design of the current disclosure according to the invention includes a PICC with at least one flushing lumen, and a valve positioned within the at least one flushing lumen. The valve comprises two configurations, an open configuration, allowing fluid flow through the distal end face opening, and a closed configuration, blocking the fluid flow through the distal end face opening. At the closed configuration of the valve, flushing fluid that is inserted into the catheter from the proximal opening is circulated back though the proximal portion and out of the subject's blood vessel, preventing the exiting of the flushing fluid though the distal end face opening. The disclosed device allows draining of the circulated flushing fluids outside of the blood vessel and/or body organ. Thus, the current disclosure enables further analysis of the flushing fluid for identification of contaminations, bacteria, and other undesired substances. Further, the valve enables controllable and rapid transitions between the open and the closed configurations, providing a closed circulating system for a bidirectional and circulated flow of the flushing fluid, therefore significantly decreasing the risk of infections and accumulation of deposits inside the catheter. The closure of the PICC, when not operational, may also assist in prevention of dwelling bacteria caused by backpressure and movement of the PICC inside the body.

Certain embodiments of the present disclosure may include some, all, or none of the above advantages. One or more other technical advantages may be readily apparent to those skilled in the art from the figures, descriptions, and claims included herein. Moreover, while specific advantages have been enumerated above, various embodiments may include all, some, or none of the enumerated advantages.

According to the invention, the at least one flushing lumen is divided into at least two individual lumens, a first lumen configured to deliver the flushing fluid, and a second lumen configured to collect the flushing fluid and direct it to the proximal portion and out of the subject's blood vessel. Thus, preventing the delivery of the flushing lumen including contaminations, precipitates, clotting factors, bacteria, and the like into the subject's body. According to some embodiments, the at least one flushing lumen may be divided into three individual lumens, wherein a third lumen is configured to deliver a medical substance to the subject.

According to some embodiments, the valve may comprise an inflatable element configured to inflate into dimension sufficient for sealing the distal end face opening. According to some embodiments, the inflatable element may be a balloon. According to some embodiments, the catheter may include an inflation conduit in fluid flow communication with the inflatable element. According to some embodiments, inflation and deflation of the inflatable element may be performed via the inflation conduit.

According to some embodiments, the valve may include a sealing flap and/or a shutter configured to controllably seal the distal end face opening.

According to some embodiments, the catheter may include one or more sensors for monitoring and/or regulating pressure and/or capacity of fluids (e.g., flushing fluid, medical substances) entering and/or exiting the catheter.

Also disclosed herein is a method for flushing of a PICC, this method not forming part of the invention as claimed. The method includes providing a PICC, as previously disclosed, and inserting the distal position of the PICC into a blood vessel and/or body organ of a subject. The method also includes shifting the valve to a closed configuration for blocking fluid flow through the distal end face opening, and injecting flushing fluid to the catheter from the proximal opening thereof, thereby the flushing fluid circulates back through the proximal portion and out of the catheter such that exiting of flushing fluid through the distal end face opening and into the subject's blood vessel is prevented.

According to some embodiments, the method further includes analyzing the flushing fluid for chemical and/or biological substances indicative of complications and/or infections. According to some embodiments, analyzing of the flushing fluid may include identification of blood clots and/or bacteria.

According to some embodiments, the method may include analyzing one or more parameters associated with a pressure of the flushing fluid exiting and/or entering the catheter for assessing the performance quality of the catheter and/or the flushing fluid.

According to some embodiments, the method may include monitoring and/or regulating pressure of the flushing fluid using one or more sensors.

According to some embodiments, the method may include monitoring and/or regulating flow capacity of the medical substance and/or flushing fluid entering the blood vessel using one or more sensors.

According to some embodiments, the method may include regulating, utilizing a controller, pressure and/or flow capacity of the flushing fluid and/or the medical substance.

According to the invention there is provided a peripherally insertable central catheter (PICC) including a distal portion, configured to be inserted into a blood vessel, the distal portion includes a distal end face opening, a proximal portion including a proximal opening, at least one flushing lumen extended therebetween, and a valve positioned within the at least one flushing lumen, the valve includes two configurations, an open configuration, allowing fluid flow through the distal end face opening, and a closed configuration, blocking fluid flow through the distal end face opening, wherein at a closed configuration of the valve, flushing fluid that is inserted to the catheter from the proximal opening thereof, circulates back through the proximal portion and out of the subject's blood vessel, thereby exiting of flushing fluid through the distal end face opening is prevented.

According to some embodiments not falling under the invention as claimed there is provided a method for flushing a peripherally insertable central catheter (PICC), the method including: providing a PICC including a distal portion including a distal end face opening, a proximal portion including a proximal opening, at least one flushing lumen extended therebetween, and a valve positioned within the at least one flushing lumen, inserting the distal portion of the PICC into a blood vessel of a subject, shifting the valve to a closed configuration for blocking fluid flow through the distal end face opening, and injecting flushing fluid to the catheter from the proximal opening thereof, thereby the flushing fluid circulates back through the proximal portion and out of the catheter such that exiting of flushing fluid through the distal end face opening and into the subject's blood vessel is prevented.

According to some embodiments, the at least one flushing lumen is further configured to deliver a medical substance to the subject at an open configuration of the valve.

According to the invention, the at least one flushing lumen is divided into at least two individual lumens, a first lumen configured to deliver the flushing fluid and a second lumen configured to collect the flushing fluid and direct it to the proximal portion and out of the subject's blood vessel.

According to some embodiments, the valve includes an inflatable element configured to inflate into dimensions sufficient for sealing the distal end face opening.

According to some embodiments, the catheter further includes an inflation conduit in fluid flow communication with the inflatable element.

According to some embodiments, the valve includes a sealing flap and/or a shutter configured to controllably seal the distal end face opening.

According to some embodiments, the valve includes a duckbill valve configured to controllably seal the distal end face opening.

According to some embodiments, the valve may include a skirt valve configured to controllably seal the distal end face opening.

According to some embodiments, the valve may include a guidewire valve configured to controllably seal the distal end face opening. According to some embodiments, the guidewire valve is a valve operated by a guidewire such that upon pushing the guidewire distally the guidewire valve is shifted to a closed configuration, thereby sealing a distal end face a PICC, and upon pulling the guidewire proximately the guidewire valve is shifted to an open configuration, thereby allowing fluid flow through a distal opening of the distal end face of the PICC.

According to some embodiments, the valve may include a piston configured to controllably seal the distal end face opening. According to some embodiments, the piston may be operated by a wire such that upon pushing the wire distally the piston is shifted into a closed configuration, thereby sealing a distal end face a PICC, and upon pulling the piston proximately the piston is shifted to an open configuration, thereby allowing fluid flow through a distal opening of the distal end face of the PICC.

According to some embodiments, the catheter further includes an additional individual lumen for delivering a medical substance to the subject at an open configuration of the valve.

According to some embodiments, the flushing fluid circulated back through the proximal portion exits the catheter through the proximal opening.

According to some embodiments, the flushing fluid circulated back through the proximal portion exits the catheter through a proximal drain port.

According to some embodiments, the catheter further includes a pressure adjustment mechanism configured to regulate a flow rate of the flushing fluid to facilitate circulation of the flushing fluid through the at least one flushing lumen, back through the proximal portion and out of the catheter.

According to some embodiments, the catheter includes a flushing lumen, a lumen for operating the valve, a lumen for delivering medical substance, and a lumen including a duckbill valve for facilitating the separation of the medical substances,

According to one aspect of the invention, the catheter further includes one or more sensors for monitoring and/or regulating pressure of the flushing fluid to facilitate pressure regulation.

According to a further aspect of the invention, the catheter further includes one or more sensors for monitoring and/or regulating flow capacity of the flushing fluid entering and/or exiting the catheter.

According to a further aspect of the invention, the catheter further includes one or more sensors for monitoring and/or regulating flow capacity of the medical substance entering the blood vessel.

According to some embodiments, the method further includes analyzing the flushing fluid for chemical and/or biological substances indicative of complications and/or infections.

According to some embodiments, the analyzing the flushing fluid includes identification of blood clots and/or bacteria.

According to some embodiments, the method further includes analyzing one or more parameters associated with a pressure of the flushing fluid exiting and/or entering the catheter for assessing the performance quality of the catheter and/or the flushing fluid.

According to some embodiments, the method further includes monitoring and/or regulating pressure of the flushing fluid using one or more sensors.

According to some embodiments, the method further includes monitoring and/or regulating flow capacity of the flushing fluid entering and/or exiting the catheter using one or more sensors.

According to some embodiments, the method further includes monitoring and/or regulating flow capacity of the medical substance entering the blood vessel using one or more sensors.

According to some embodiments, the method further includes regulating, utilizing a controller, pressure and/or flow capacity of the flushing fluid and/or the medical substance.

Unless otherwise defined, all technical and scientific terms used herein have the same meaning as commonly understood by one of ordinary skill in the art to which this disclosure pertains. In case of conflict, the patent specification, including definitions, governs. As used herein, the indefinite articles "a" and "an" mean "at least one" or "one or more" unless the context clearly dictates otherwise.

### BRIEF DESCRIPTION OF THE FIGURES

Some embodiments of the disclosure are described herein with reference to the accompanying figures. The description, together with the figures, makes apparent to a person having ordinary skill in the art how some embodiments may be practiced. The figures are for the purpose of illustrative description and no attempt is made to show structural details of an embodiment in more detail than is necessary for a fundamental understanding of the disclosure. For the sake of clarity, some objects depicted in the figures are not drawn to scale. Moreover, two different objects in the same figure may be drawn to different scales. In particular, the scale of some objects may be greatly exaggerated as compared to other objects in the same figure.

In block diagrams and flowcharts, optional elements/components and optional stages may be included within dashed boxes.

In the figures:
**FIG. 1** shows a schematic illustration of a prior art PICC introduced into a subject's blood vessel;
**FIG. 2** schematically shows a prior art PICC;
**FIG. 3** schematically shows a side view of a distal portion of a PICC with a valve, according to some embodiments;
**FIG. 4A** schematically shows a perspective view of a distal portion of a PICC, according to some embodiments;
**FIG. 4B** schematically shows a cross-section view of the distal portion and the distal end face of the PICC of **Fig. 4A****,** according to some embodiments;
**FIG. 5A** schematically shows a perspective view of a distal portion of a PICC in an open valve configuration, according to some embodiments;
**FIG. 5B** schematically shows a perspective view of the distal portion of the PICC of **Fig. 5A****,** in closed valve configuration, according to some embodiments;
**FIG. 6A** schematically shows a cross-section side view of a distal portion of a PICC in an open valve configuration, according to some embodiments;
**FIG. 6B** schematically shows a cross-section side view of a distal portion of a PICC in a closed valve configuration, according to some embodiments;
**FIG. 7A** schematically shows a cross-section side view of a distal portion of a PICC in an open valve configuration, according to some embodiments;
**FIG. 7B** schematically shows a cross-section side view of a distal portion of a PICC in a closed valve configuration, according to some embodiments;
**FIG. 8A** schematically shows a cross-section side view of a distal portion of a PICC in an open valve configuration, according to some embodiments;
**FIG. 8B** schematically shows a cross-section side view of a distal portion of a PICC in a closed valve configuration, according to some embodiments;
**FIG. 9A** schematically shows a cross-section side view of a distal portion of a PICC in an open valve configuration, according to some embodiments;
**FIG. 9B** schematically shows a cross-section side view of a distal portion of a PICC in a closed valve configuration, according to some embodiments; and
**FIG. 10** shows a flow chart of a method of flushing of a PICC, according to some embodiments.

### DETAILED DESCRIPTION

The principles, uses and implementations of the teachings herein may be better understood with reference to the accompanying description and figures. Upon perusal of the description and figures present herein, one skilled in the art will be able to implement the teachings herein without undue effort or experimentation. In the figures, same reference numerals refer to same parts throughout.

In the following description, various aspects of the invention will be described. For the purpose of explanation, specific details are set forth in order to provide a thorough understanding of the invention. However, it will also be apparent to one skilled in the art that the invention may be practiced without specific details being presented herein. Furthermore, well-known features may be omitted or simplified in order not to obscure the invention.

The present invention relates to peripherally inserted central catheters (PICC).

The disclosed PICC, according to some embodiments, is configured to deliver a medical substance to the subject at an open valve configuration. The medical substance may include drugs, such as cytotoxic drugs for cancer treatment, antibiotic medicines, blood, nutrition, and the like.

According to some embodiments, the disclosed PICC may by implemented for medical substance delivery, optionally for a simultaneous delivery of several medical substances, for preventing undesired mixing of the medical substances before entering the blood vessel and/or body organ. Following the delivery of the medical substances, flushing of the PICC may by performed. An aspect of some embodiments of the present invention relates to a catheter including a PICC having a distal portion, a proximal portion, a valve, and at least one flushing lumen extended in between. The valve, positioned within the at least one flushing lumen, has at least two configurations, an open configuration, allowing fluid flow through the distal end face opening, and a closed configuration, blocking fluid flow through the distal end face opening. Thus, in the closed valve configuration, the fluid inserted through the proximal end face opening is circulated back and exits the catheter through the proximal drain port. The exiting fluid, e.g., the circulated fluid, may be further analyzed for identification of bacteria, clotting factors and contaminations, for assessing the catheter function and/or the subject's medical condition.

According to some embodiments, the at least one flushing lumen may be a lumen within which fluid may flow in at least one direction between the distal end face opening and the proximal drain port. According to some embodiments, the at least one flushing lumen may be configured to enable fluid flow in at least two directions between the distal end face opening and the proximal drain port. According to some embodiments, the at least one flushing lumen may include a plurality of individual lumens. According to some embodiments, the at least one flushing lumen may be divided into a plurality of individual lumens. According to some embodiments, the at least one flushing lumen may be divided into a plurality of individual lumens, wherein a first lumen may be configured to deliver the flushing fluid, and a second lumen may be configured to receive the flushing fluid and direct it to the proximal portion and out of the subject's blood vessel and/or body organ. According to some embodiments, the plurality of individual lumens may be in fluid communication.

According to some embodiments, the at least one flushing lumen includes a valve. According to some embodiments, the valve may include any element configured to prevent entering and/or exiting of fluids from the PICC assembly to the blood vessel and/or the vice versa.

According to some embodiments, the valve positioned within the at least one flushing lumen, may be located inside the distal portion inside of the PICC. According to some embodiments, the valve may be positioned at the distal end face of the PICC. According to some embodiments, and as described in greater detail elsewhere herein, the valve may be positioned between a cavity defined by closed configuration of the valve, an inner wall of the PICC, and the distal end of the plurality of individual lumens, allowing circulating of fluids inside the cavity at the distal portion of PICC, and outside the plurality of individual lumens. Following the circulation, the fluids (e.g., flushing fluids) are directed back towards the proximal end and out of the catheter-outside the subject's body.

According to some embodiments, the valve may include an inflatable element, such as a balloon, configured to inflate into dimensions sufficient for sealing the distal end face opening, thus preventing fluid flow into a blood vessel and/or body organ. According to some embodiments, sealing of the PICC may require the inflatable element to be fully or partially inflated (when in closed configuration). According to some embodiments, the inflatable element, when in closed (inflated) configuration, may be positioned fully and/or partially inside, or fully outside the distal end face of the PICC. According to some embodiments, the valve may include a ring located on the outer and/or inner circumference (e.g., external and/or internal ring) of the at least one flushing lumen, wherein the ring is configured to shift between open and closed configurations. For example, the ring may govern the closed configuration by reducing the circumference of the at least one flushing lumen until no fluid can enter and/or exit the distal end face of the PICC assembly; the ring may govern the open configuration by increasing the circumference of the flushing lumen, allowing entering and/or exiting fluids through the distal end face. According to some embodiments, the at least one flushing lumen may comprise a groove configured to retain the ring, providing a smooth outer surface of the flushing lumen. A smooth external and/or internal surface area is beneficial for minimizing the accumulation of undesired substances and deposits on the external and/or internal surface areas of the lumen, for decreasing the risk of infections, bacteria growth, and various contaminations.

According to some embodiments, the distal portion of the PICC assembly may include an angular distal end face. The angular distal end face may include a conus-like shape, wherein the diameter of the at least one flushing lumen is decreasing at the distal end face. The valve may be located at the conus-like shape of the distal end face, wherein the valve is configured to shift from the open and the closed configurations (and vice versa), as required. Valve configuration may be governed mechanically, for example, by applying a mechanical pressure positioning and retaining the valve at the narrowest region of the conus-like configuration, e.g., at the angular distal end face.

According to some embodiments, the valve may include a sealing flap and/or a shutter, configured to controllably seal the distal end face opening. Preferably, the sealing will occur rapidly, minimizing leakage from and/or into the catheter. Optionally, a service conduit may be permanently positioned at the at least one flushing lumen, for example at a service lumen, at the distal portion of the PICC. Optionally or alternatively, the service conduit may be temporarily positioned inside the at least one flushing lumen, and thus may be removed following the shifting of the valve configuration. The service conduit may be elongated, reaching the proximal end face of the PICC assemble, thereby allowing an external control of the valve configuration, for example, for controlling and/or applying mechanical pressure on the valve. According to some embodiments, service conduit positioned inside service lumen may prevent fluid communication of the service lumen with the at least one flushing lumen of the PICC. Put differently, when the service lumen is associated with the service conduit (e.g., for inflation/deflation of an inflatable element by a gas flow) no fluids may enter and/or exit the service lumen.

According to some embodiments, the service conduit may be associated with the valve, wherein sealing of the distal end face is performed by reducing the diameter of the at least one flushing lumen until no gap is present at the distal end face, preventing penetration of fluids.

According to some embodiments, the catheter may include a pressure adjustment mechanism, configured to regulate and/or monitor a flow rate of the flushing fluid to facilitate circulation of the flushing fluid through the at least one flushing lumen. Furthermore, since the volume of the administrated fluids is relatively high with respect to the surface area of the catheter-a long and a thin lumen-pressure adjustment mechanism may be needed for providing the optimal power flow. The pressure adjustment mechanism may include, for example, a pump and/or pumping system for the entering and/or exiting fluids.

According to some embodiments, the catheter may include a valve, e.g., a balloon valve, a flushing lumen, a service lumen, and a lumen with a duckbill valve. The flushing lumen may be utilized for nutrition supplements delivery and/or for flushing purposes, the service lumen may be utilized for the valve configuration operating, and the lumen with the duckbill valve may be applied for administrating e.g., cytotoxic drugs to the subject. The duckbill valve may also include any other type of a controlled valve for regulating the entering and/or exiting fluids, and for facilitating the separation of the medical substances and minimizing residue formation inside the catheter. Moreover, the operating mechanism of the duckbill valve is technically simple, thus it may be beneficial for the subject to use such a catheter, allowing an independent operating that does not require presence at a medical facility.

Optionally, in some embodiments, the catheter may include one or more sensors, allowing pressure regulation and/or monitoring of the flushing fluid, thereby facilitating pressure regulation inside the lumens of the catheter. In some embodiments, one or more sensors may regulate and/or monitor the flow capacity of the flushing fluid and/or the medical substances entering and/or exiting the catheter.

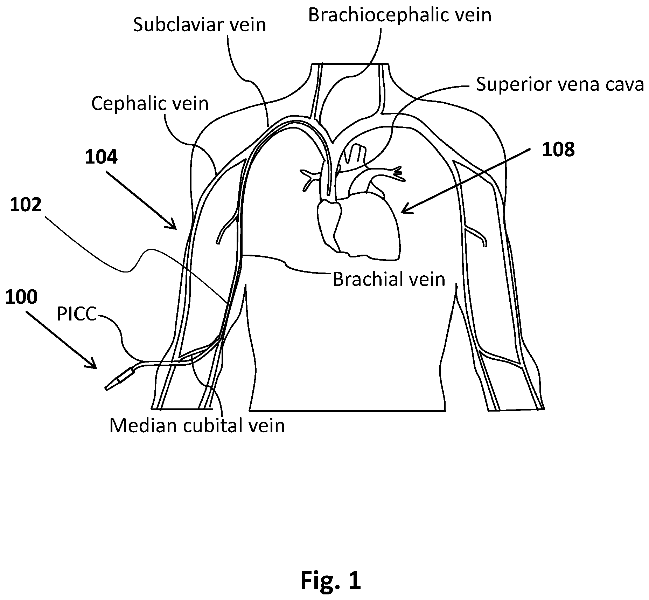

Reference is now made to **Fig. 1****,** which schematically shows a peripherally insertable central catheter (PICC) **100** introduced into a blood vessel **102** of a subject, according to prior art. PICC **100** is shown herein positioned in blood vessel **102,** which is positioned in an upper arm **104** of the subject, is configured to deliver medical substance (not shown) through blood vessel **102** into a body organ, such as heart **108.**

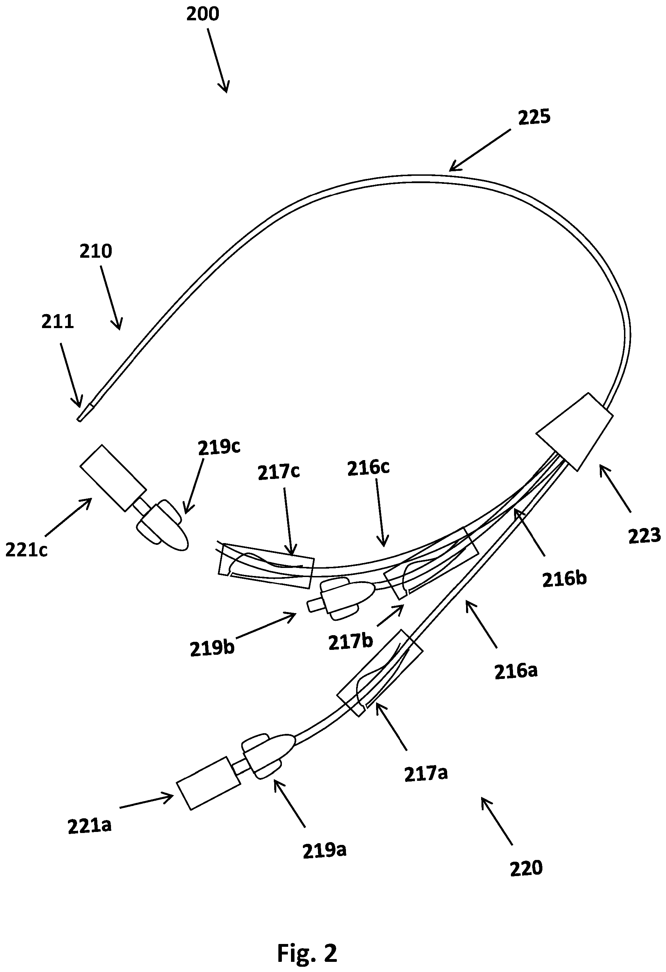

Reference is now made to **Fig. 2****,** which schematically shows a standard PICC **200,** according to prior art. Standard PICC **200** has a distal portion **210,** configured to be inserted into a blood vessel. Optionally, distal portion **210** may include a soft tip **211,** for minimizing trauma risk of the blood vessel. A proximal portion **220** of the PICC **200** may optionally include three individual lumens, lumen **216a, 216b** and **216c.** Each of lumens **216a, 216b** and **216c** is associated with an adapter **223** configured to connect a catheter line **225** with each of the lumens **216a, 216b** and **216c.** Each of lumens **216a, 216b** and **216c** is equipped with each of clamps **217a, 217b** and **217c,** respectively, configured to allow a temporarily sealing (e.g., for several minutes, hours or days) at the proximal end **220** without removing PICC **200** from the blood vessel. Further, each of lumens **216a, 216b** and **216c** may be equipped with each of connectors **219a, 219b** and **219c.** Connectors **219a, 219b** and **219c** may be needleless connectors, allowing a temporarily association with IV and/or syringes and/or external tubes or lumens, configured to deliver medical substances and/or other fluids (e.g., flushing fluid) into PICC **200.** As shown in **Fig. 2****,** connectors **219a** and **219c** may be associated with removable caps **221a** and **221c** for reducing infection risks. For example, a syringe (not shown) may be associated with lumen **216a** via connector **219a** at the proximal portion of the PICC **200.** Then, upon opening clamp **217,** a medical substance from the syringe may be administered into PICC **200** by flowing through adapter **223,** catheter line **225** and exiting into the blood vessel via distal portion **210.** Following the administration of medical substances, and especially in scenarios where administration of several types of medical substances is required, flushing of the catheter is performed for removing contaminations, bacteria, and the like. Flushing procedure is usually performed by inserting a flushing fluid, such as a saline solution, into one of the lumens (e.g., lumen **216c**) at the proximal end **220** of PICC **200.** Upon reaching the distal portion **210,** the flushing fluid, including the contaminations, clotting factors, bacteria, and the like, is delivered into the blood vessel and/or body organ of the subject. Disposing the contaminations into the subject leads to an increased risk of infections and health problems.

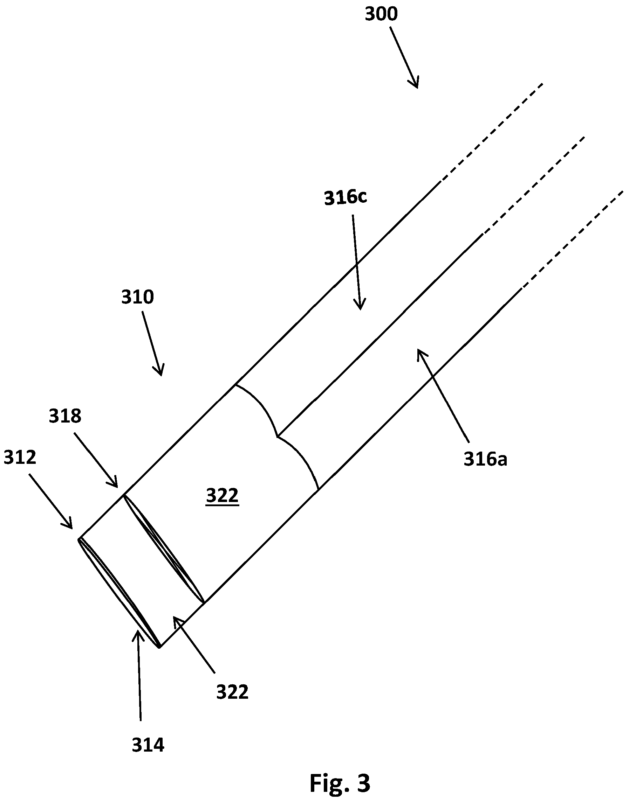

Reference is now made to **Fig. 3****,** which shows a schematic illustration of a distal portion **310** of a PICC **300,** according to some embodiments. PICC **300** is designed to facilitate circulation of flushing fluid such that the drained fluid disposal into a body organ is prevented. Hence, may limit, reduce, or eliminate bacteria, contaminations and/or precipitates which are administered from the flushing fluid into the subject. According to some embodiments of the current disclosure, distal portion **310** includes a distal end face **312,** a distal opening **314,** individual lumens **316a** and **316c,** and a valve **318.** Lumens **316a** and **316c** extend along PICC **300** from a proximal portion thereof (not shown) to distal portion **310.** As seen in **Fig. 3****,** lumens **316a** and **316c** do not reach all the way to distal end face **312** forming a cavity **322** defined by an inner wall of PICC **300,** the distal end of lumens **316a** and **316c** and valve **318.** According to some embodiments, valve **318** is positioned inside cavity **322.** According to some embodiments, flushing fluid may be inserted from a proximal end (not shown) of PICC **300** through lumen **316a,** then, when valve **318** is in a closed configuration, the flushing fluid is circulated at distal portion **310** and drained from a proximal end (not shown) outside of PICC 300 through lumen **316c.** According to some embodiments, flushing fluid may be inserted through the proximal end (not shown) of lumen **316c,** circulated at distal portion **310,** and then drained from a proximal end (not shown) outside of PICC **300** through lumen **316a.** In other embodiments, flushing fluid may be drained out from each of lumens **316a** and **316c** and out of the body.

According to some embodiments, a cross-sectional size (e.g., for example, diameter) of lumen **316a** may be different and/or equal to a cross-sectional size of the lumen **316c.** According to some embodiments, the cross-sectional sizes of the lumens **316a/316c** may correlate with a flow rate of fluid flowing therein. According to some embodiments, the cross-sectional sizes of the lumens **316a/316c** may be different. According to some embodiments, the cross-sectional sizes of the lumens **316a/316c** may vary. According to some embodiments, different configurations in which lumens **316a** and **316c** have different cross-sectional sizes may induce pressure differences between lumens **316a** and **316c,** thereby enabling a desirable flow rate in each of the lumens.

According to some embodiments, a wall thickness of each of the lumens **316a/316c** may be different. According to some embodiments, the wall thickness of at least one of the lumens **316a/316c** may vary. Advantageously, having different and/or varying wall thicknesses for the lumens **316a/316c** may allow desirable pressure differences of fluid flow therein. According to some embodiments, having different and/or varying wall thicknesses for the lumens **316a/316c** may increase the durability of the PICC.

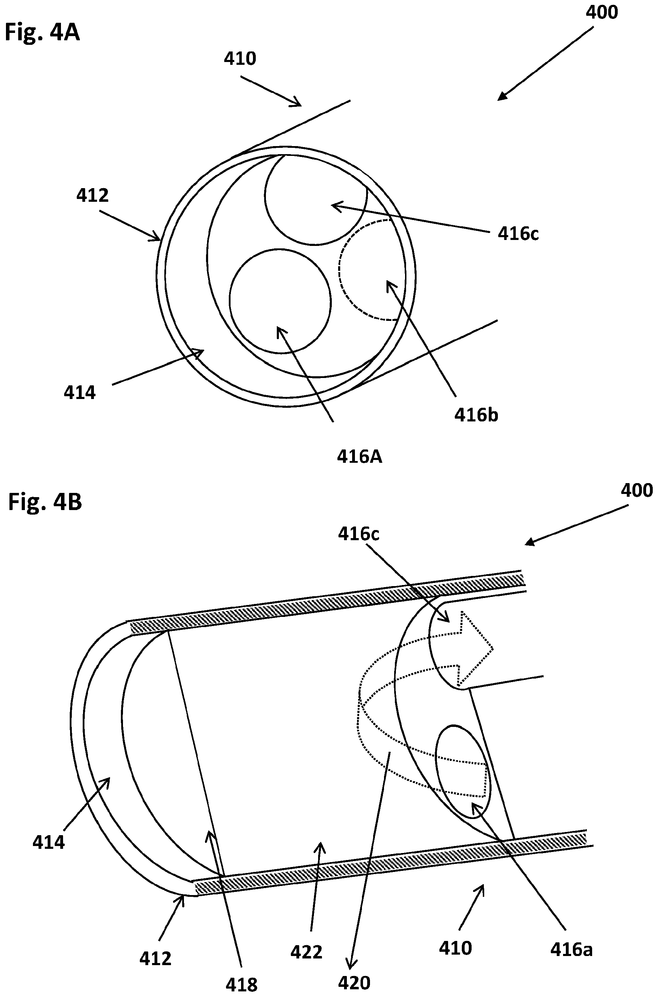

Reference is now made to **Fig. 4A** and **Fig 4B****,** which show a schematic illustration of a perspective view of a distal portion **410** of a PICC **400** and show a cross-section view of distal portion **410** and distal end face **412** of PICC **400,** according to some embodiments. Distal portion **410** includes a distal opening **414** located at a distal end face **412** of PICC **400.** PICC **400** includes an individual lumen **416a,** an individual lumen **416c,** and, optionally, may also include an individual lumen **416b.** According to some embodiments, lumen **416b** may include one or more individual lumens. Lumens **416a, 416b** and **416c** extend along PICC **400** from a proximal portion thereof (not shown) to distal portion **410.** As also seen in **Fig. 3****,** lumens **416a, 416b** and **416c** do not reach all the way to distal end face **412** forming a cavity **422** defined by an inner wall of PICC **400,** the distal end of lumens **416a, 416b** and **416c** and a valve **418** (as depicted in **Fig. 4B**). According to some embodiments, lumen **416a** may be utilized as a medical substance administration rout, and lumens **416a** and **416c** may be utilized as flushing lumens, while the lumen **416b** may be utilized as the service lumen for distal valve (such as valve **418,** as depicted in **Fig. 4B**) operating. In other words, lumen **416a** may serve both as a medical substance administrator and as a flushing lumen. According to some embodiments, each of lumens **416a-c** may, independently, be implemented as the medical substance administrator, and/or as the flushing lumen and/or as the service lumen. According to some embodiments where the service lumen is present (such as lumen **416b**), it may be advantageous to utilize the service lumen for valve operating only.

Reference is now made to **Fig. 4B****,** which shows a schematic illustration of a cross-sectional view of distal portion **410** of **PICC 400** depicted in **Fig. 4A****,** according to some embodiments. According to some embodiments, PICC **400** includes a plurality of individual lumens, an individual lumen **416a** and an individual lumen **416c.** **Fig. 4B** schematically depicts a flow direction **420** of a flushing fluid introduced, for example, into lumen **416a** from the proximal end face of PICC **400** (not shown). As depicted by **Fig. 4B****,** closed configuration of a valve **418** results in circulation of the flushing fluid, due to a flow of fluid from lumen **416a** into lumen **416c.** The fluid from lumen **416a** flows from the proximal end of the PICC towards the valve **418** (and/or towards the distal end of the PICC). The valve **418,** at a closed configuration thereof, constricts the flow of the fluid from lumen **416a** from exiting the distal end of the PICC, thereby forcing the circulation of the fluid into lumen **416c,** as depicted by arrow **420.** Consequently, the closed configuration of valve **418** prevents entering of the flushing fluid into the blood vessel/body organ, and allows draining of the flushing fluid outside of the blood vessel and/or body organ. Hence, reducing the risk of infections and other medical complications.

According to some embodiments, the plurality of individual lumens may include one or more valves, such as duckbill valves (not shown). The one or more valves are configured to prevent entering of the circulated fluid into the distal end of one or more of the individual lumens and flow towards the proximal end therein. As a non-limiting example, a PICC **400** may include a plurality of lumens, such as four individual lumens. A first lumen **416a** (i.e., flushing lumen) configured to deliver the flushing fluid, a second lumen **416c** (i.e., flushing lumen) configured to receive the flushing fluid and direct it to the distal end and outside the subject, and a lumen **416b** which includes two individual lumens, a first individual lumen (not shown) optionally configured to operate a valve **418,** and a second individual lumen (not shown), which includes the one or more valves and, optionally, configured to deliver medical substance which should not be mixed with other substances and/or fluids. When valve **418** shifts from open to closed configuration, flushing fluid entering the first lumen **416a** is circulated inside a cavity **422,** i.e., the flushing fluid changes the flow direction from the distal portion **410** towards the proximal portion (not shown) of PICC. Hence, to avoid entering of the circulated fluid into the second individual lumen, the second individual lumen may include the one or more valves. According to some embodiments, the one or more valves may be positioned inside the second individual lumen. According to some embodiments, each of the plurality of lumens may include one or more valves.

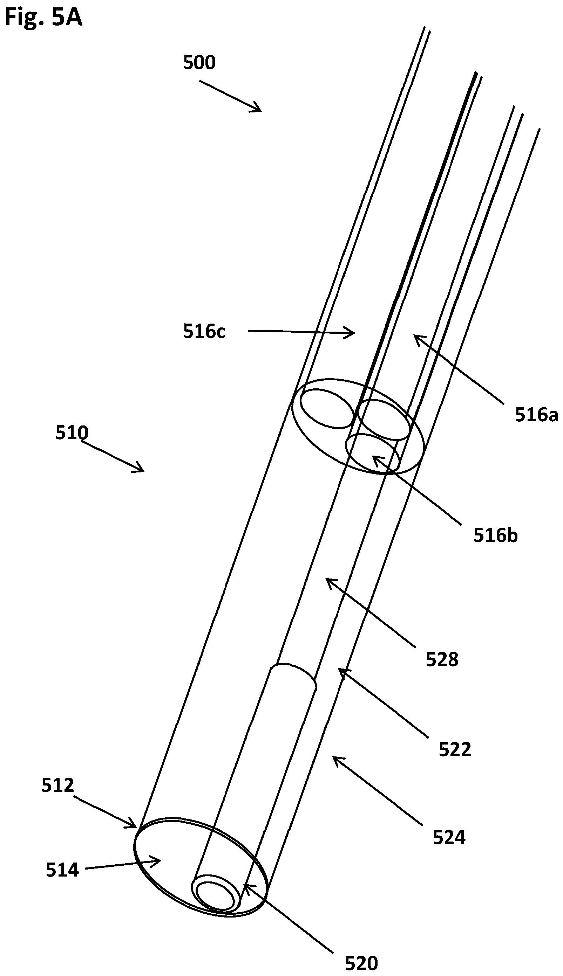

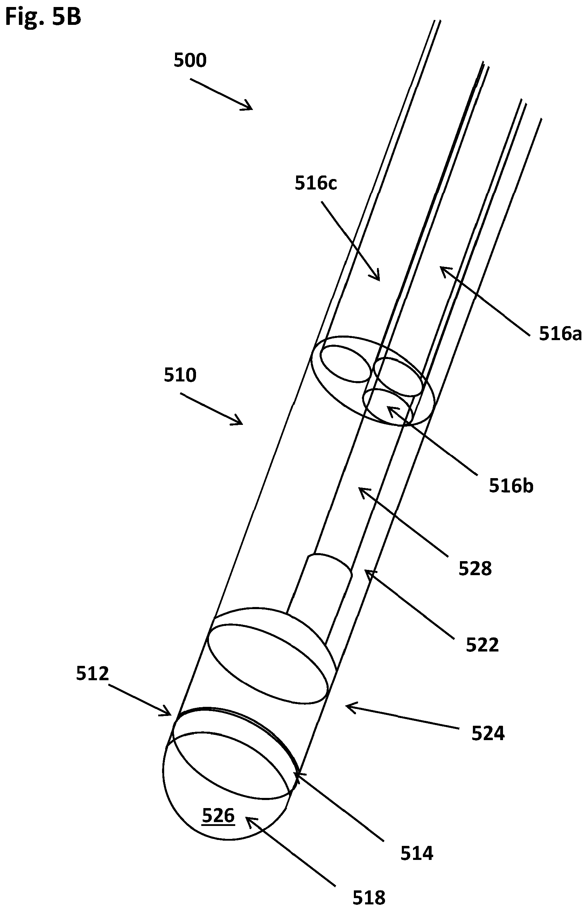

Reference is now made to **Figs. 5A** and **5B****,** which show a schematic perspective view of a distal portion **510** of a PICC **500** in an open valve configuration **520** (depicted in **Fig. 5A**) and in a closed valve configuration **518** (depicted in **Fig. 5B**), according to some embodiments. Distal portion **510** includes a distal opening **514** located at a distal end face **512** of PICC **500.** According to some embodiments, PICC **500** includes an individual lumen **516a,** an individual lumen **516b** and an individual lumen **516c,** a cavity **522** formed between the distal end of the lumens **516b** and **516c** and the distal end face **512** of PICC **500,** and a valve **524** positioned inside the cavity **522.** According to some embodiments, lumen **516c** may serve as a service lumen and may include a service conduit **528.** In some embodiments, service conduit **528** may control valve **522** configuration shifting (e.g., from closed valve configurating **518** to open valve configuration **520,** and vice versa). In some embodiments, service conduit **528** may assist during administration of PICC **500** into a blood vessel and/or body organ. For example, while catheters are typically made of soft materials which are not visible at X-ray imaging, service conduit **528** may be made of materials that assist viability and stability during X-ray, thus facilitating easier and proper positioning of PICC **500** inside a blood vessel and/or body organ. As depicted in **Fig. 5A****,** the open valve configuration **520** allows administrating of medical substance into the blood vessel and/or body organ of the subject.

As depicted in **Fig. 5B****,** the valve **524** may include a balloon **526.** According to some embodiments, at a closed configuration **518,** the valve **524** (or the balloon **526**) may be at least partially inflated. According to some embodiments, at a closed configuration **518,** the valve **524** may be inflated such that the balloon **526** obstructs the distal end of the PICC. According to some embodiments, the PICC may include a service conduit **528** configured to deliver fluid (e.g., for example, a gas) to and/or from the balloon **526.** According to some embodiments, the service conduit **528** may be in fluid communication with the balloon **526.** According to some embodiments, the service conduit **528** may be positioned within the lumen **516c.** According to some embodiments, the service conduit **528** may be positioned on an outer wall and/or abutting the PICC. Advantageously, an inflated balloon valve may prevent the flushing fluid from exiting the distal opening **514** and entering the blood vessel and/or body organ. Advantageously, when the PICC is idle and the balloon **526** of valve **524** is inflated, it may prevent infections dwelling back from the body to one or more lumens.

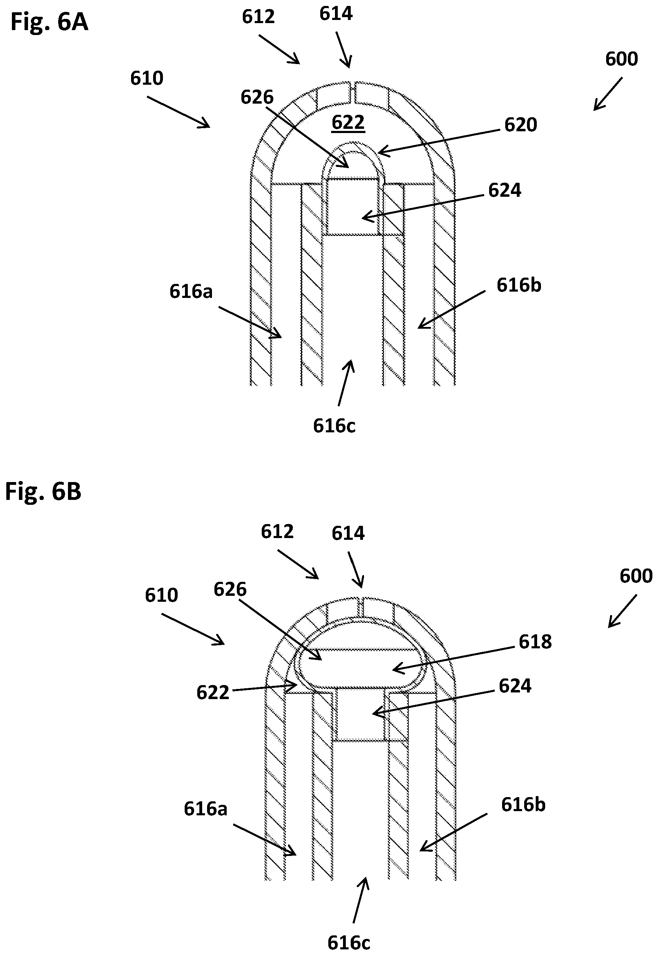

Reference is now made to **Figs. 6A** and **6B****,** which show a schematic cross-sectional side view of a distal portion **610** of a PICC **600** in an open valve configuration **620** (depicted in Fig. **6A**) and in a closed valve configuration **618** (depicted in **Fig. 6B**), according to some embodiments.

According to some embodiments, the following components/features depicted in **Figs. 6A** and **6B** **600, 610, 612, 614, 616a-c, 622** and **624** correspond to and may have identical or similar structure, configuration and/or characteristics as the previously described herein components **500, 510, 512, 514, 516a-c, 522** and **524** in **Fig 5****.**

According to some embodiments and as depicted in **Figs. 6A** and **6B****,** a distal portion **610** of the PICC **600** may include, among others, a circular or circular-like structure. Alternatively, the distal portion **610** of the PICC **600** may include an angle-shaped structure, such as but not limited to, conus-like, rectangular, polynomic structure, and the like or any combination thereof. According to some embodiments, PICC **600** includes an individual lumen **616a,** an individual lumen **616b** and an individual lumen **616c.** According to some embodiments, PICC **600** may include 2 individual lumens (e.g., individual lumens **616a** and **616b**). According to some embodiments, PICC **600** may include 3 or more individual lumens. Each possibility is a separate embodiment.

According to some embodiments, PICC **600** includes a cavity **622** formed between a distal end of the individual lumens **616ba, 616b** and **616c** and a distal end face **512** of PICC **600,** and a valve **624** positioned inside the cavity **622.** According to some embodiments, the valve **624** includes an inflatable element, such as a balloon **626** configured to inflate into dimensions sufficient for sealing a distal opening **614,** thus preventing fluid flow into and/or from a blood vessel and/or body organ. According to some embodiments, the PICC **600** may include an inflation conduit (e.g., in the individual lumen **616c**) in fluid flow communication with the inflatable element. According to some embodiments, inflation and deflation of the inflatable element may be performed via the inflation conduit. According to some embodiments, the balloon **626** may be inflated with fluid, such as but not limited to, saline solution. According to some embodiments, the inflation of the balloon **626** may be performed at a proximal portion (not shown) of the PICC **600.**

According to some embodiments and as depicted in **Figs. 6A** and **6B****,** the balloon **626** is partly positioned within the cavity **622** and partially inside the individual lumen **616c.** According to some embodiments, the balloon **626** may be positioned outside the individual lumen **616c.** According to some embodiments, the balloon **626** may be positioned outside the individual lumen **616c.** As a non-limiting example, the balloon **626** may be positioned on an outer portion of the individual lumen **616c.** As yet another non-limiting example, the balloon **626** may be essentially positioned within the cavity **622.**

According to some embodiments and as depicted in **Fig. 6B****,** the closed valve configuration **618** of the balloon **626** includes at least a partial inflation thereof, essentially filling the cavity **622,** thereby preventing fluid from there though. Additionally or alternatively, in some embodiments, the closed valve configuration **618** may include filling the cavity **626** as well as filling a distal portion of each of the individual lumens **616a** and **616b.**

According to some embodiments, the closed valve configuration **618** includes at least partial inflation of the balloon **626** extending outside the distal end face **612.** According to some embodiments, the closed valve configuration **618** the balloon **626** is inflated by occupying only the inter components of the PICC **600** (e.g., by filling the cavity **622**).

According to some embodiments, the individual lumen **616c** is a service lumen configured to control and operate the configuration shifting (i.e. open/closed) of the valve **624.** According to some embodiments, the individual lumen **616c** is a service lumen configured to control the inflated/deflated configuration of the balloon **626.** According to some embodiments, PICC **600** may include or be associated with a service conduit positioned inside the individual lumen **616c.** According to some embodiments, the service conduit may be in fluid communication with the valve **624.** According to some embodiments, the service conduit may control the configuration shifting of the valve **624** (e.g., from closed valve configuration **618** to open valve configuration **620,** and vice versa). In some embodiments, the service conduit may assist in administration of PICC **600** into a blood vessel and/or body organ.

According to some embodiments, the valve shifting mechanism may be controlled and/or performed manually, e.g., by a caregiver and/or by the subject by mechanically operating the valve **624** at a proximal end of the PICC **600.** Alternatively or additionally the valve shifting mechanism may be performed automatically, for example, by sensors control.

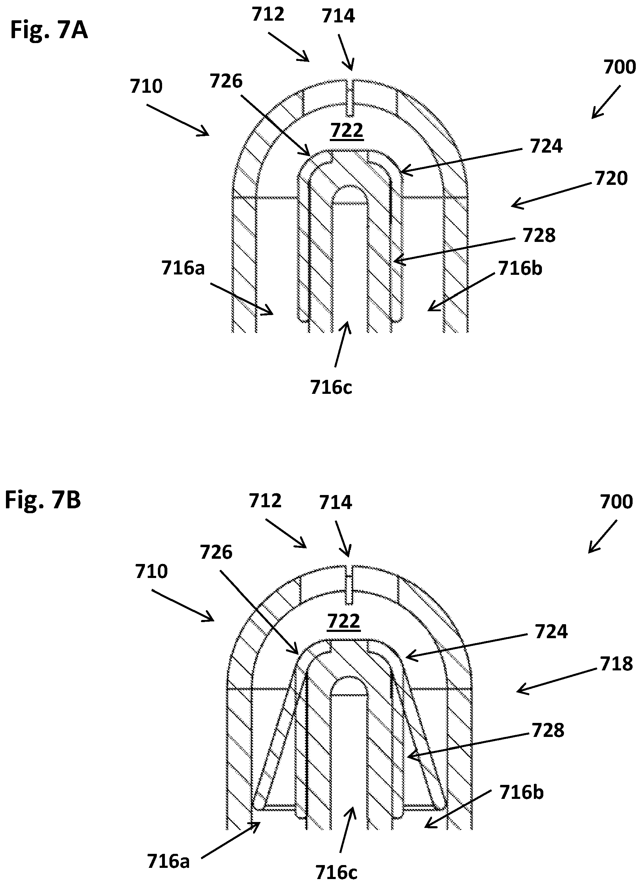

Reference is now made to **Figs. 7A** and **7B****,** which show a schematic cross-sectional side view of a distal portion **710** of a PICC **700** in an open valve configuration **720** (depicted in Fig. **7A**) and in a closed valve configuration **718** (depicted in **Fig. 7B**), according to some embodiments.

According to some embodiments, the following components/features depicted in **Figs. 7A** and **7B** **700, 710, 712, 714** and **716a-c,** correspond to and may have identical or similar structure, configuration and/or characteristics as the previously described herein components **600, 610, 612, 614** and **616a-c** in **Figs 6A** and **6B****.**

According to some embodiments, a cavity **722** is formed between a distal end of an individual lumens **716a** and **716c** and a distal end face **712** of PICC **600,** and a valve **724** positioned inside the cavity **722.**

According to some embodiments, the valve **724** includes a sealing flap and/or a shutter configured to controllably seal the distal end face opening. According to some embodiments, the sealing flap and/or the shutter may be in a form of a skirt valve **726.**

According to some embodiments, the valve **724** includes a skirt valve **726** configured to controllably seal a distal opening **714.** According to some embodiments, the skirt valve **726** may be fused or otherwise integrally formed on a distal end of the outer surface of the individual lumen **716c.** According to some embodiments, the skirt valve **726** may be detachably associated with the individual lumen **716c.** According to some embodiments, the skirt valve **726** includes a rim **728,** such that in the open valve configuration **720** the rim **728** is essentially vertically positioned, allowing fluid flow therethrough, and in the closed valve configuration **718** the rim **728** is in a downwardly sloping position, thereby preventing passing of fluids therethrough. Alternatively or additionally, in some embodiments, the closed valve configuration **718** may include an upwardly sloping position. According to some embodiments and as depicted in **Fig. 7B****,** sealing of the PICC **900** by the skirt valve **826** includes an internal sealing of each of the individual lumens **716a and 716b.** According to some embodiments, the closed configuration **718** of the skirt valve **726** may include sealing the cavity **722.**

According to some embodiments, without wishing to be bound to any specific mechanism, the skirt valve **726** may be operated via a service conduit (not shown) positioned within the individual lumen **716c.** As a non-limiting example, the service conduit may be associated, among others, with a wire such that upon pushing the wire distally the rim **728** is shifted into the downwardly sloping position (i.e. the closed configuration **718**), sealing the distal end face **712** of the PICC **700,** and upon pulling the wire proximately the rim **728** is shifted into an essentially vertical position, (i.e. the open configuration **720),** thereby allowing fluid flow through a distal opening of the distal end face of the PICC.

According to some embodiments, the configuration of the skirt valve **726** may be mechanically or manually (e.g., by a subject and/or a caregiver) operated from a proximal end of the PICC **700,** and/or automatically operated, for example, by sensors control. As a non-limiting example, the skirt valve **726** configuration may be controlled by operating a service conduit (not shown) positioned within the individual lumen **716c.**

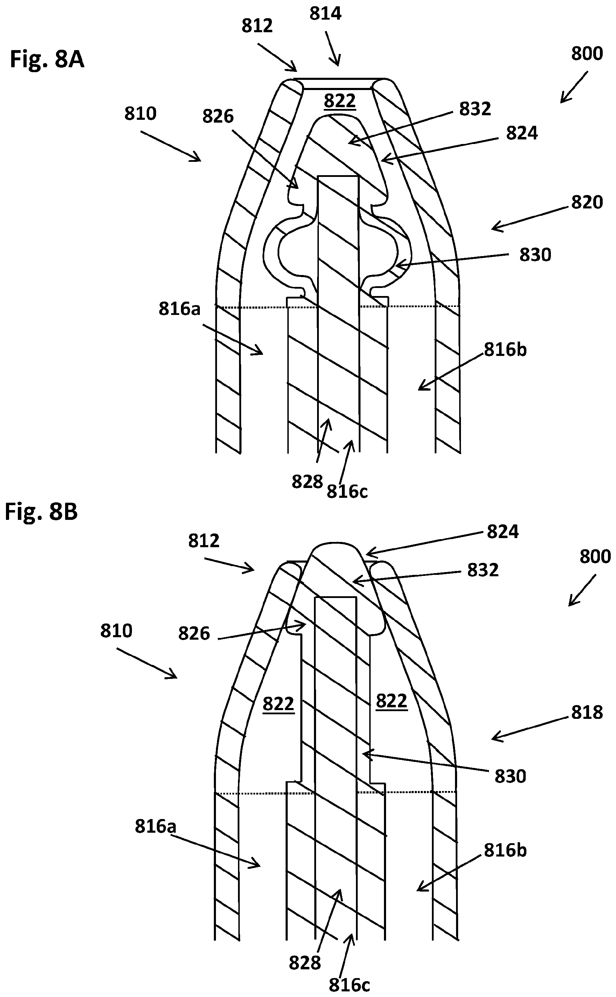

Reference is now made to **Figs. 8A** and **8B****,** which show a schematic cross-sectional side view of a distal portion **810** of a PICC **800** in an open valve configuration **820** (depicted in Fig. **8A**) and in a closed valve configuration **818** (depicted in **Fig. 8B**), according to some embodiments.

According to some embodiments and as depicted in **Figs. 8A** and **8B****,** a distal portion **810** of the PICC **800** includes an angle-shaped structure, such as but not limited to, conus-like structure. Alternatively, in some embodiments, the distal portion **810** may include a circular or circular-like structure. According to some embodiments, PICC **800** includes an individual lumen **816a,** an individual lumen **816b** and an individual lumen **816c.** According to some embodiments, the individual lumen **816c** is a service lumen. According to some embodiments, PICC **800** may include two individual lumens. According to some embodiments, PICC **800** may include three or more individual lumens. Each possibly is a separate embodiment.

According to some embodiments, a cavity **822** is formed between a distal end of the individual lumens **816a, 816b** and **816c** and a distal end face **812** of PICC **800,** and a valve **824** is positioned inside the cavity **822.**

According to some embodiments, the valve **824** includes a guidewire valve **826** configured to controllably seal a distal opening **814.** According to some embodiments, the guidewire valve **826** includes a cap **832,** an elastic valve member **830,** and is detachably or permanently associated with a guidewire **828.**

According to some embodiments, the guidewire valve **826** is a valve operated by the guidewire **828** such that upon pushing the guidewire **828** distally the guidewire valve **826** is in the closed configuration **820,** thereby sealing the distal end face **812** of the PICC **800,** and upon pulling the guidewire **828** proximately the guidewire valve **826** is in the open configuration **818,** thereby allowing fluid flow through the distal opening **814** of the distal end face **812.**

According to some embodiments, the guidewire valve **826** may be fused or otherwise integrally formed on the outer surface of a distal end of the individual lumen **816c.** As a non-limiting example, at least a portion of the elastic valve member **830** may be associated with the distal end of the individual lumen **816c.** According to some embodiments, the guidewire valve **826** may be detachably associated with the individual lumen **816c.**

According to some embodiments, the guidewire valve **826** may be inserted into the PICC **800** only when sealing thereof is required. Alternatively, in some embodiments, the guidewire valve **826** may be positioned within the PICC both at open and closed configurations **818** and **820,** respectively, thereof.

According to some embodiments, the guidewire valve **826** includes or made of flexible, elastic and/or soft materials, such as but not limited to, polymers. According to some embodiments, the cap **832** and the elastic member **830** may include or made of different materials than the guidewire **828.** According to some embodiments, the guidewire **828** may include or be made of materials having lower flexibility relative to the cap **832** and/or the elastic member **830.** According to some embodiments, the guidewire **828** may include or be made of metals, such as but not limited to, aluminum.

According to some embodiments, the cap **832** is configured to seal the distal end face **812** of the PICC **800,** thus preventing fluid flow therethrough According to some embodiments, the structure of the cap **832** is geometrically complementary with the distal opening **814** and/or with at least a portion of the distal end face **812.** According to some embodiments, the cap **832** includes, among others, an angular structure (e.g., pyramid-like, conus-like, and the like) to facilitate sealing the distal end face **812** of the PICC **800.**

According to some embodiments, the elastic valve member **830** is positioned on the outer portion of a lumen, e.g., the individual lumen **816c.** Alternatively or additionally, in some embodiments, at least a portion of the elastic valve member **830** may be positioned inside the individual lumen **816c.** According to some embodiments, the guidewire valve **826** is associated with a guidewire **828** positioned inside the individual lumen **816c,** allowing shifting the configuration of the guidewire valve **826.**

According to some embodiments and as depicted in **Fig. 8A****,** in the open valve configuration, the elastic valve member **830** is at least partially folded, allowing passage of fluids through the distal opening **814** of the distal end face **812** of the PICC **800.** According to some embodiments, in the open valve configuration **818** the cap **832** is at least partially positioned inside the cavity **822.** According to some embodiments and as depicted in **Fig. 8B****,** in the closed valve configuration, the elastic valve member **830** is pushed distally (e.g., by extending, unfolding and the like of the elastic member **830**) such that the cap **832** seals the distal end face **812** of the PICC **800.**

According to some embodiments, the configuration of the guidewire valve **826** may be mechanically operated from a proximal end of the PICC **800,** and/or automatically operated, for example, by sensors control. According to some embodiments, implementing the guidewire valve **826** advantageously allows technically simple operation of the PICC **800** and reduces clogging risks.

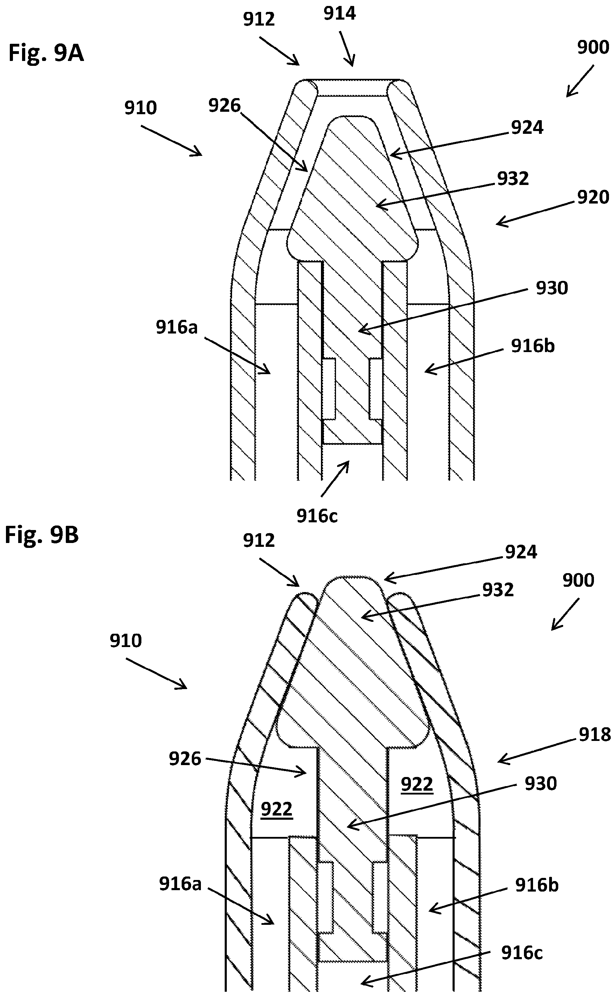

Reference is now made to **Figs. 9A** and **9B****,** which show a schematic cross-sectional side view of a distal portion **910** of a PICC **900** in an open valve configuration **920** (depicted in Fig. **9A**) and in a closed valve configuration **918** (depicted in **Fig. 9B**), according to some embodiments.

According to some embodiments, the following components/features depicted in **Figs. 9A** and **9B** **900, 910, 912, 914** and **916a-c,** correspond to and may have identical or similar structure, configuration and/or characteristics as the previously described herein components **800, 810, 812, 814** and **816a-c** in **Figs 8A** and **8B****.**

According to some embodiments, a cavity **922** is formed between a distal end of individual lumens **916a, 916b** and **916c** and a distal end face **912** of PICC **900,** and a valve **924** is positioned inside the cavity **922.**

According to some embodiments, the valve **924** is includes a piston **926** configured to controllably seal a distal opening **914.** According to some embodiments and as depicted in **Figs. 9A** and **9B****,** the piston **926** is partially positioned within the individual lumen **916c.** According to some embodiments, the piston **926** includes or made of rigid or semi-rigid materials, such as but not limited to, polymers, metals, and the like.

According to some embodiments, the piston **926** may be operated by a wire or any other suitable pushing means (not shown) such that upon pushing the wire distally the piston **926** is shifted to the closed configuration **920,** thereby sealing a distal end face **912** of the PICC **900,** and upon pulling the wire proximately the piston **926** is shifted to the open configuration **918,** thereby allowing fluid flow through the distal opening **914** of the distal end face **912.**

According to some embodiments, the piston **926** includes a body **930** and a cap **932.** According to some embodiments, at least a portion of the body **930** of the piston **926** is configured to fit inside a lumen e.g., a service lumen, e.g., the individual human **916c.** According to some embodiments, the piston **926** is advantageously positioned within the individual lumen **916c,** minimizing the risks of infections, clogging, bacteria accumulation, and the like. According to some embodiments, the cap **932** includes an angular structure, such as but not limited to, a pyramid, conus, and the like, configured to seal the distal end face **912** of the PICC **900.** According to some embodiments, the cap **932,** in the closed configuration **918,** is at least partially extended from the distal end face **912.** According to some embodiments, the piston **926** operation is advantageously independent of flushing pressure within the individual lumen **916c.**

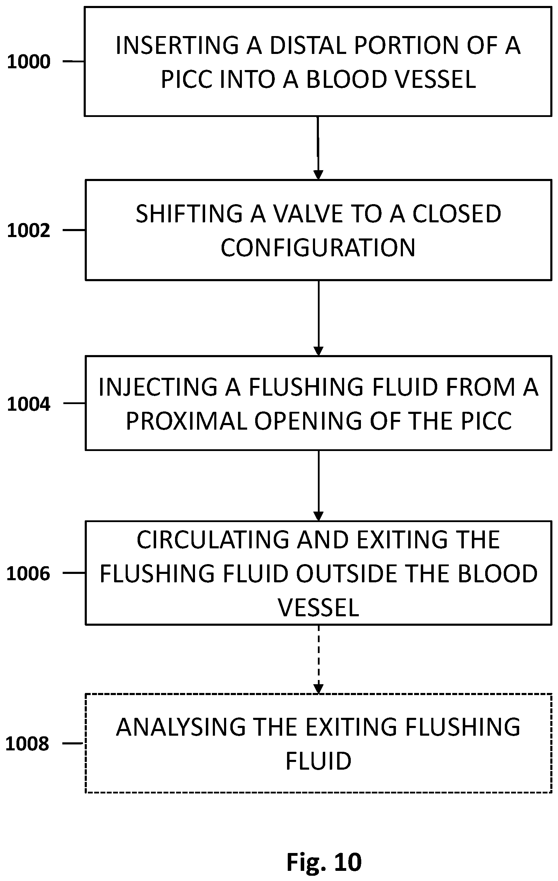

According to some embodiments, the piston **926** is at least partially positioned within the individual lumen **916c** at the open and/or the closed configurations **920** and **918,** respectively, thereof. According to some embodiments, the piston **926** advantageously provides operational certainty (e.g., by minimizing inadvertent pushing/pulling or otherwise displacing thereof) of each of the open and the closed configurations **920** and **918,** respectively. Reference is now made to **Fig. 10****,** which shows a flow chart of a method of flushing of a PICC, according to some embodiments, such as but not limited to, PICCs **300, 400** and **500.**

At step **1000,** the method includes inserting a distal portion of a PICC into a blood vessel. According to some embodiments, a caregiver inserts a distal portion (such as distal portion **310, 410, 510, 610, 710, 810** or **910** of the PICC **300, 400, 500, 600, 700, 800** or **900**) into a blood vessel and/or organ of a subject. Optionally, the inserting may be performed by using a guidewire, e.g., nitinol guidewire, and/or by imaging devices, such as X-ray, to validate the proper locating of a PICC inside the subject.

At step **1002,** the method includes shifting a valve to a closed configuration. According to some embodiments, the valve located at the distal portion of the PICC is shifted to a closed configuration. According to some embodiments, valve may include, but is not limited to, an inflatable balloon, which, when inflated, seals the distal portion of the PICC. Valve shifting mechanism may be controlled manually, e.g., by a caregiver and/or the subject by mechanically operating the valve at a proximal end, and/or automatically, for example, by sensors control.

At step **1004,** the method includes injecting a flushing fluid from a proximal opening of the PICC. According to some embodiments, the flushing fluid, such as saline, may be inserted into the catheter from the proximal opening.

At step **1006,** the method includes circulating and exiting the flushing fluid outside the blood vessel. According to some embodiments, the flushing fluid continues to flow until reaching the distal portion of the PICC. Upon reaching the closed valve configuration, flow direction of the flushing fluid changes-circulates, and the flushing fluid enters a different lumen, allowing the flushing fluid flow from the distal portion to the proximal portion of the catheter and outside the body. In some embodiments, the flushing fluid may be circulated and enter the same lumen.

At step **1008,** the method includes analysing the exiting flushing fluid. According to some embodiments, upon exiting of the flushing fluid, the flushing fluid may be collected, and optionally, further analysed for presence of any chemical and/or biological substances, such as, for example, contaminations, precipitates, bacteria, and the like. According to some embodiments, presence of chemical and/or biological substances may be indicative of complications and/or infections. According to some embodiments, analysis of the exiting flushing fluid allows identification of blood clots and/or bacteria. According to some embodiments, identification of bacteria presence may assist the medical staff in providing proper treatment to the subject, thereby increasing health status and wellness of the subject.

According to some embodiments, the method may include analyzing one or more parameters associated with a pressure of the flushing fluid exiting and/or entering the catheter for assessing the performance quality of the catheter and/or the flushing fluid, as well as may assist with measuring the quantities of medical substance administered. In some embodiments, exceeding the maximum allowed pressure inside the PICC leads to formation of various defects, such as kinks, which may decrease the integrity of the PICC. According to some embodiments, pressure changes inside the PICC may indicate presence of precipitates and/or accumulation of residue. Accumulation of undesired materials and obstructions inside the PICC may eventually lead to blockage of the PICC. According to some embodiments, the method may include monitoring pressure of the medical substance and/or flushing fluid using one or more sensors. One or more sensors may be located at various positions across the length of the PICC, for example, at the distal end and/or the proximal end. According to some embodiments, one or more sensors may be associated with one or more of the lumens. According to some embodiments, the one or more sensors may be configured to monitor the pressure of the medical substance and/or flushing fluid within one or more of the lumens. In some embodiments, it may be preferable to place the one or more sensors such that a smooth inner and/or outer surface of the lumens and/or the PICC is maintained, thereby reducing a risk of infections.

According to some embodiments, the method may include monitoring flow capacity of the medical substance entering the blood vessel using one or more sensors. According to some embodiments, changes in flow capacity may indicate malfunction of the PICC, and, optionally, may require a replacement of the PICC.

According to some embodiments, the method may include monitoring flow capacity of the flushing fluid entering and/or exiting the catheter using one or more sensors. According to some embodiments, changes in the flow capacity of the flushing fluid may indicate valve malfunction and/or replacement. According to some embodiments, valve replacement may be performed by a service conduit.

According to some embodiments, the method may include regulating, utilizing a controller, pressure and/or flow capacity of the flushing fluid and/or the medical substance. Consequently, facilitating the maintaining of pressure and/or flow capacity in the optimal range, thus increasing integrity of the PICC.

In the description and claims of the application, the words "include" and "have", and forms thereof, are not limited to members in a list with which the words may be associated.

Unless otherwise defined, all technical and scientific terms used herein have the same meaning as commonly understood by one of ordinary skill in the art to which this disclosure pertains. In case of conflict, the patent specification, including definitions, governs. As used herein, the indefinite articles "a" and "an" mean "at least one" or "one or more" unless the context clearly dictates otherwise.

It is appreciated that certain features of the disclosure, which are, for clarity, described in the context of separate embodiments, may also be provided in combination in a single embodiment. Conversely, various features of the disclosure, which are, for brevity, described in the context of a single embodiment, may also be provided separately or in any suitable sub-combination or as suitable in any other described embodiment of the disclosure. No feature described in the context of an embodiment is to be considered an essential feature of that embodiment, unless explicitly specified as such.

Although stages of methods according to some embodiments may be described in a specific sequence, methods of the disclosure may include some or all of the described stages carried out in a different order. A method of the disclosure may include a few of the stages described or all of the stages described. No particular stage in a disclosed method is to be considered an essential stage of that method, unless explicitly specified as such.

Although the disclosure is described in conjunction with specific embodiments thereof, it is evident that numerous alternatives, modifications and variations that are apparent to those skilled in the art may exist.

It is to be understood that the disclosure is not necessarily limited in its application to the details of construction and the arrangement of the components and/or methods (not claimed) set forth herein. Other embodiments may be practiced, and an embodiment may be carried out in various ways.

The phraseology and terminology employed herein are for descriptive purpose and should not be regarded as limiting.

Section headings are used herein to ease understanding of the specification and should not be construed as necessarily limiting.

## Claims

1. A peripherally insertable central catheter (PICC) (300) comprising
a distal portion (310), configured to be inserted into a blood vessel, the distal portion comprises a distal end face opening (314);
a proximal portion comprising a proximal opening;
at least one flushing lumen extended therebetween, the at least one flushing lumen is divided into at least two individual lumens, a first lumen (316a) configured to deliver the flushing fluid and a second lumen (316c) configured to collect the flushing fluid and direct it to the proximal portion and out of the subject's blood vessel; and
a valve (318) positioned within the at least one flushing lumen, the valve comprises two configurations, an open configuration, allowing fluid flow through the distal end face opening, and a closed configuration, blocking fluid flow through the distal end face opening, wherein at a closed configuration of the valve, flushing fluid that is inserted to the catheter from the proximal opening thereof, circulates back through the proximal portion and out of the subject's blood vessel, thereby exiting of flushing fluid through the distal end face opening is prevented; and
one or more sensors for:
monitoring and/or regulating pressure of the flushing fluid to facilitate pressure regulation and/or
monitoring and/or regulating flow capacity of the flushing fluid entering and/or exiting the catheter; and/or
monitoring and/or regulating flow capacity of the medical substance entering the blood vessel.

2. The catheter of claim 1, wherein the at least one flushing lumen is further configured to deliver a medical substance to the subject at an open configuration of the valve.

3. The catheter of claim 1, wherein the valve comprises an inflatable element configured to inflate into dimensions sufficient for sealing the distal end face opening.

4. The catheter of claim 3, further comprising an inflation conduit in fluid flow communication with the inflatable element.

5. The catheter of claim 1, wherein the valve comprises a sealing flap and/or a shutter configured to controllably seal the distal end face opening.

6. The device of any one of claims 1-5, wherein the valve comprises a guidewire valve, a piston, a duckbill valve or a skirt valve configured to controllably seal the distal end face opening.

7. The catheter of any one of claims 1-6, further comprising an additional individual lumen for delivering a medical substance to the subject at an open configuration of the valve.

8. The catheter of any one of claims 1-7, wherein the flushing fluid circulated back through the proximal portion exits the catheter through the proximal opening.

9. The catheter of any one of claims 1-8, wherein the flushing fluid circulated back through the proximal portion exits the catheter through a proximal drain port.

10. The catheter of any one of claims 1-9, further comprising a pressure adjustment mechanism configured to regulate a flow rate of the flushing fluid to facilitate circulation of the flushing fluid through the at least one flushing lumen, back through the proximal portion and out of the catheter.

11. The catheter of claim 1, comprising a flushing lumen, a lumen for operating the valve, a lumen for delivering medical substance, and a lumen comprising a duckbill valve for facilitating the separation of the medical substances.

## Patentansprüche

1. Peripher einführbarer Zentralkatheter (PICC) (300), umfassend
einen distalen Abschnitt (310), der dazu konfiguriert ist, in ein Blutgefäß eingeführt zu werden, wobei der distale Abschnitt eine distale Stirnflächenöffnung (314) umfasst;
einen proximalen Abschnitt, der eine proximale Öffnung umfasst;
mindestens ein Spüllumen, das sich dazwischen erstreckt, wobei das mindestens eine Spüllumen in mindestens zwei einzelne Lumina unterteilt ist, ein erstes Lumen (316a), das dazu konfiguriert ist, das Spülfluid zuzuführen, und ein zweites Lumen (316c), das dazu konfiguriert ist, das Spülfluid zu sammeln und es zu dem proximalen Abschnitt und aus dem Blutgefäß des Subjekts heraus zu leiten; und
ein Ventil (318), das innerhalb des mindestens einen Spüllumens angeordnet ist, wobei das Ventil zwei Konfigurationen umfasst, eine offene Konfiguration, die eine Fluidströmung durch die distale Stirnflächenöffnung ermöglicht, und eine geschlossene Konfiguration, die eine Fluidströmung durch die distale Stirnflächenöffnung blockiert, wobei in einer geschlossenen Konfiguration des Ventils Spülfluid, das von der proximalen Öffnung des Katheters aus in den Katheter eingeführt wird, zurück durch den proximalen Abschnitt und aus dem Blutgefäß des Subjekts heraus zirkuliert, wodurch ein Austreten von Spülfluid durch die distale Stirnflächenöffnung verhindert wird; und
einen oder mehrere Sensoren zum:
Überwachen und/oder Regulieren des Drucks des Spülfluids, um eine Druckregulierung zu erleichtern, und/oder
Überwachen und/oder Regulieren der Durchflusskapazität des in den Katheter eintretenden und/oder aus dem Katheter austretenden Spülfluids; und/oder
Überwachen und/oder Regulieren der Durchflusskapazität der in das Blutgefäß eintretenden medizinischen Substanz.

2. Katheter nach Anspruch 1, wobei das mindestens eine Spüllumen ferner dazu konfiguriert ist, dem Subjekt eine medizinische Substanz in einer offenen Konfiguration des Ventils zuzuführen.

3. Katheter nach Anspruch 1, wobei das Ventil ein aufblasbares Element umfasst, das dazu konfiguriert ist, sich auf Abmessungen aufzublasen, die zum Abdichten der distalen Stirnflächenöffnung ausreichen.

4. Katheter nach Anspruch 3, ferner umfassend eine Aufblasleitung, die in Fluidströmungsverbindung mit dem aufblasbaren Element steht.

5. Katheter nach Anspruch 1, wobei das Ventil eine Dichtungsklappe und/oder eine Abdeckung umfasst, die dazu konfiguriert sind, die distale Stirnflächenöffnung steuerbar abzudichten.

6. Vorrichtung nach einem der Ansprüche 1 bis 5, wobei das Ventil ein Führungsdrahtventil, einen Kolben, ein Entenschnabelventil oder ein Schürzenventil umfasst, die dazu konfiguriert sind, die distale Stirnflächenöffnung steuerbar abzudichten.

7. Katheter nach einem der Ansprüche 1 bis 6, ferner umfassend ein zusätzliches einzelnes Lumen zum Zuführen einer medizinischen Substanz zu dem Subjekt in einer offenen Konfiguration des Ventils.

8. Katheter nach einem der Ansprüche 1 bis 7, wobei das Spülfluid, das zurück durch den proximalen Abschnitt zirkuliert, durch die proximale Öffnung aus dem Katheter austritt.

9. Katheter nach einem der Ansprüche 1 bis 8, wobei das Spülfluid, das zurück durch den proximalen Abschnitt zirkuliert, durch einen proximalen Ablassport aus dem Katheter austritt.

10. Katheter nach einem der Ansprüche 1 bis 9, ferner umfassend einen Druckeinstellmechanismus, der dazu konfiguriert ist, eine Durchflussrate des Spülfluids zu regulieren, um eine Zirkulation des Spülfluids durch das mindestens eine Spüllumen, zurück durch den proximalen Abschnitt und aus dem Katheter heraus zu erleichtern.

11. Katheter nach Anspruch 1, umfassend ein Spüllumen, ein Lumen zum Betätigen des Ventils, ein Lumen zum Zuführen medizinischer Substanz und ein Lumen, das ein Entenschnabelventil zum Erleichtern der Trennung der medizinischen Substanzen umfasst.

## Revendications

1. Cathéter central insérable par voie périphérique (PICC) (300) comprenant
une partie distale (310), configurée pour être insérée dans un vaisseau sanguin, la partie distale comprenant une ouverture de la face d'extrémité distale (314) ;
une partie proximale comprenant une ouverture proximale ;
au moins une lumière de rinçage s'étendant entre celles-ci, ladite au moins une lumière de rinçage étant divisée en au moins deux lumières individuelles, une première lumière (316a) configurée pour délivrer le fluide de rinçage et une deuxième lumière (316c) configurée pour collecter le fluide de rinçage et le diriger vers la partie proximale et hors du vaisseau sanguin du sujet ; et
une valve (318) positionnée à l'intérieur de ladite au moins une lumière de rinçage, la valve comprenant deux configurations, une configuration ouverte, permettant l'écoulement de fluide à travers l'ouverture de la face d'extrémité distale, et une configuration fermée, bloquant l'écoulement de fluide à travers l'ouverture de la face d'extrémité distale, dans lequel, dans une configuration fermée de la valve, le fluide de rinçage qui est inséré dans le cathéter depuis l'ouverture proximale dudit cathéter circule en retour à travers la partie proximale et hors du vaisseau sanguin du sujet, de sorte que la sortie de fluide de rinçage à travers l'ouverture de la face d'extrémité distale est empêchée ; et
un ou plusieurs capteurs pour :
surveiller et/ou réguler la pression du fluide de rinçage afin de faciliter la régulation de la pression et/ou
surveiller et/ou réguler la capacité d'écoulement du fluide de rinçage entrant dans le cathéter et/ou sortant du cathéter ; et/ou
surveiller et/ou réguler la capacité d'écoulement de la substance médicale entrant dans le vaisseau sanguin.

2. Cathéter selon la revendication 1, dans lequel ladite au moins une lumière de rinçage est en outre configurée pour délivrer une substance médicale au sujet dans une configuration ouverte de la valve.

3. Cathéter selon la revendication 1, dans lequel la valve comprend un élément gonflable configuré pour se gonfler jusqu'à des dimensions suffisantes pour obturer l'ouverture de la face d'extrémité distale.

4. Cathéter selon la revendication 3, comprenant en outre un conduit de gonflage en communication d'écoulement de fluide avec l'élément gonflable.

5. Cathéter selon la revendication 1, dans lequel la valve comprend un clapet d'obturation et/ou un volet configurés pour obturer de manière contrôlée l'ouverture de la face d'extrémité distale.

6. Dispositif selon l'une quelconque des revendications 1 à 5, dans lequel la valve comprend une valve à fil-guide, un piston, une valve à bec de canard ou une valve à jupe configurés pour obturer de manière contrôlée l'ouverture de la face d'extrémité distale.

7. Cathéter selon l'une quelconque des revendications 1 à 6, comprenant en outre une lumière individuelle supplémentaire pour délivrer une substance médicale au sujet dans une configuration ouverte de la valve.

8. Cathéter selon l'une quelconque des revendications 1 à 7, dans lequel le fluide de rinçage ayant circulé en retour à travers la partie proximale sort du cathéter à travers l'ouverture proximale.

9. Cathéter selon l'une quelconque des revendications 1 à 8, dans lequel le fluide de rinçage ayant circulé en retour à travers la partie proximale sort du cathéter à travers un orifice de drainage proximal.

10. Cathéter selon l'une quelconque des revendications 1 à 9, comprenant en outre un mécanisme d'ajustement de la pression configuré pour réguler un débit du fluide de rinçage afin de faciliter la circulation du fluide de rinçage à travers ladite au moins une lumière de rinçage, en retour à travers la partie proximale et hors du cathéter.

11. Cathéter selon la revendication 1, comprenant une lumière de rinçage, une lumière pour actionner la valve, une lumière pour délivrer de la substance médicale, et une lumière comprenant une valve à bec de canard pour faciliter la séparation des substances médicales.
